# EUROPÄISCHE PATENTSCHRIFT

(11) **EP 1 235 906 B1**
(45) Veröffentlichungstag und Bekanntmachung des Hinweises auf die Patenterteilung: **11.04.2007**
(21) Anmeldenummer: 00987254.0
(22) Anmeldetag: 16.11.2000
(51) Int. Cl.: C12N 15/00, A01H 1/06, C12N 15/70, C12N 15/74, C12N 15/29, C12N 15/82, C12N 1/21, C12N 5/10, A01H 11/00

(54) **VERFAHREN ZUR MUTAGENESE VON NUKLEOTIDSEQUENZEN AUS PFLANZEN, ALGEN, ODER PILZEN**
METHOD FOR THE MUTAGENESIS OF NUCLEOTIDE SEQUENCES FROM PLANTS ALGAE OR FUNGI
PROCEDE DE MUTAGENESE DE SEQUENCES NUCLEOTIDIQUES DE PLANTES, D'ALGUES OU DE CHAMPIGNONS

(30) Priorität: 26.11.1999 EP 99123611
(43) Veröffentlichungstag der Anmeldung: 04.09.2002
(73) Patentinhaber: BASF Plant Science GmbH, 67056 Ludwigshafen (DE); Universitätsklinikum Freiburg, 79104 Freiburg (DE)
(72) Erfinder: RAK, Bodo, 79106 Freiburg (DE); RESKI, Ralf, 79254 Oberried (DE); ZIMMERMANN, Susanne, 72072 Tübingen (DE); GUITTON, Marie-Christine, F-68500 Jungholtz (FR); DUWENIG, Elke, 67063 Ludwigshafen (DE); FREUND, Annette, 67117 Limburgerhof (DE)
(74) Vertreter: Fitzner, Uwe
(86) Internationale Anmeldenummer: PCT/EP2000/011326
(87) Internationale Veröffentlichungsnummer: WO 2001/038509

(56) Entgegenhaltungen:
- RESKI R: "Physcomitrella and Arabidopsis: the David and Goliath of reverse genetics" TRENDS IN PLANT SCIENCE, Bd. 3, Nr. 6, Juni 1998 (1998-06), Seiten 209-210, XP000881435
- STREPP R, ET AL.: "Plant nuclear gene knockout reveals a role in plastid division for the homolog of the bacterial cell division protein FtsZ, an ancestral tubulin" PROCEEDINGS OF THE NATIONAL ACADEMY OF SCIENCES OF THE USA, Bd. 95, April 1998 (1998-04), Seiten 4368-4373, XP002135451
- RESKI R: "Molecular genetics of Physcomitrella" PLANTA, Bd. 208, Nr. 3, Mai 1999 (1999-05), Seiten 301-309, XP000881434
- SCHAEFER DG, ZRYD J-P: "Efficient gene targeting in Physcomitrella patens" PLANT JOURNAL, Bd. 11, Nr. 6, Juni 1997 (1997-06), Seiten 1195-1206, XP000885764
- MENGISTE T, PASZKOWSKI J: "Prospects for the Precise Engineering of Plant Genomes by Homologous Recombination" BIOLOGICAL CHEMISTRY, Bd. 380, Nr. 7-8, Juli 1999 (1999-07) - August 1999 (1999-08), Seiten 749-758, XP000885763

## Beschreibung

Die vorliegende Erfindung betrifft ein Verfahren zur Mutagenese eukaryontischer Nukleotidsequenzen, bevorzugt Pflanzen, Algen und/oder Pilze sowie ein Verfahren zur Herstellung solcher genetisch veränderter eukaryontischer Zellen.

Ein mögliches Verfahren zur Aufklärung von Genfunktionen ist die Inaktivierung von Genen und die anschließende Beobachtung der Auswirkungen auf den Stoffwechsel oder das Erscheinungsbild des modifizierten Systems.

Insbesondere die Inaktivierung von Genen durch Insertionsmutagenese hat sich in eukaryontischen Systemen als schwierig erwiesen. Der Erfolg der Inaktivierung von Genen durch homologe Rekombination im Wirtsgenom ist wesentlich davon abhängig, in welchem Verhältnis unerwünschte, ortsunabhängige Rekombination gegenüber der ortsspezifischen homologen Rekombination auftritt. Dieses Verhältnis ist in Eukaryonten extrem unterschiedlich. Eine ausreichend hohe Effizienz der Methode zur Erzeugung von vielen Mutanten ist nur in Niederen Eukaryonten und ausgehend von genomischer DNA beschrieben. Effizienzen oberhalb von 10% konnten bisher nur in Hefen (Grimm & Kohli, 1988, MGG 215, 87-93; Struhl 1983, Nature 305, 391-397), verschiedenen filamentösen Pilzen (Fotheringham & Holloman, 1989, Mol Cell Biol 9, 4052-4055; Kronstad et al., 1989, Gene 79, 97-106; Paietta & Marzluff, 1985, Mol Cell Biol 5, 1554-1559; Timberlake & Marshall, 1989, Science 244, 1313-1317) und in Dictyostelium discoideum (De Lozanne & Spudich, 1987, Science 236, 1086-1091) gezeigt werden.

Homologe Rekombination in Pflanzen wurde bisher aus Arabidopsis thaliana (Kempin et al., Nature 1998, 389, 802-803) für das AGL5-Gen, einen MADS Box Faktor, sowie für den TGA3 Lokus berichtet (Miao & Lam 1995, Plant Journal 7, 359-365). Dabei konnten jedoch lediglich Einzelereignisse festgestellt werden, die keine Aussage über die statistische Häufigkeit zulassen (Puchta 1998, Trends Plant Sci 3 (3), 77-80). Änderungen im Phänotyp traten in diesen Fällen nicht auf. Im Fall des AGL5-Gens war eine von 750 analysierten Pflanzen ortsspezifisch mutiert (entsprechend einer Rekombinationsrate von 0,13 %), im Fall des TGA3-Lokus wurde ein Ereignis aus 2580 Mutanten gefunden (entsprechend einer Rekombinationsrate von 0,04%). Aus Lotus japonica wurde berichtet, daß aus 18974 Transformanten kein homologes Rekombinationsereignis nachgewiesen werden konnte (Thykjaer et al. 1997, Plant Mol Biol 35, 523-530).

Unter Verwendung genomischer Fragmente von Einzelkopiegenen (single copy genes) wurden aus dem Moos Physcomitrella patens "knockout"-Mutanten in Folge homologer Rekombination erhalten (Schaefer et al., 1997, Plant Journal 11 (6): 1195-1206). Die Rate homologer Rekombination lag bei 90%. Allerdings konnte auch in diesem Fall keine Änderung im Phänotyp beobachtet werden.

Die Nutzung genomischer Nukleotidsequenzen zur Erzeugung von Mutanten, ist insofern von Nachteil, als daß die DNA-Fragmente verschiedene operative Einheiten oder Verknüpfungen beinhalten können. Unter einer operativen Einheit oder Verknüpfung versteht man die sequenzielle Anordnung einzelner Bausteine und ihre Verknüpfung untereinander wie Promotor, codierender Sequenz, Terminator und ggf. weiterer regulativer Elemente derart, daß jedes der regulativen Elemente seine Funktion bei der Expression der kodierenden Sequenz bestimmungsgemäß erfüllen kann. Durch die ortsungenaue Insertion können dabei z.B. Promotoren derart zufällig modifiziert werden, daß ein Regulator mit neuen Eigenschaften entsteht.

Bei der Analyse der Mutanten können weitere Probleme auftreten. Werden z.B. zwei Gene durch ein Nukleinsäurefragment disruptiert, kann nachfolgend kein eindeutiger Rückschluß auf die Beteiligung eines Gens geschlossen werden, da der Beitrag eines einzelnen Gens am zu beobachtenden Effekt nicht bewertet werden kann. Dies müßte erst durch aufwendigere Analysen, wie beispielsweise die Sequenzierung der verwendeten genomischen Fragmente und durch Bestimmung der Art der Insertion in der Pflanze und seine Auswirkungen auf die genomische Anordnung veränderter Genabschnitte überprüft werden.

Ferner weist die Inaktivierung von Genen durch die Insertionsmutagenese genomischer DNA mittels homologer Rekombination nach bekannten Verfahren einen weiteren erheblichen Nachteil auf. So ist beispielsweise zur Analyse des gesamten Genoms eines Wirtssytems die aufwendige Konstruktion von mehreren tausend Einzelkonstrukten genomischen Ursprungs erforderlich, die ebenfalls einzeln in die Wirtszelle retransformiert werden müssen, bevor eine Analyse der nach homologer Rekombination erhaltenen Mutanten möglich wird. Solche genomischen Mutationsansätze sind somit wenig wirtschafltich und für einen routinemäßigen Durchsatz größerer Ansätze, beispielsweise mit dem Ziel einer "gesättigten" Mutagenese des gesamten Wirtsgenoms, ungeeignet.

Aufgabe der vorliegenden Erfindung ist es daher ein einfaches und effizientes Verfahren zur Mutagenese in eukaryontischen Zellen zur Verfügung zu stellen, das die zuvor genannten Nachteile nicht mehr aufweist.

Dies wird erfindungsgemäß gelöst durch ein Verfahren zur Mutagenese eukaryontischer Nukleotidsequenzen, wobei eine genomische Nukleotidsequenz und/oder cDNA-Sequenz aus Eukaryonten in einen Mikroorganismus übertragen wird, die eukaryontische Nukleotidsequenz in dem Mikroorganismus durch sequenzunabhängige Insertionsmutagenese genetisch verändert wird und anschließend diese genetisch veränderte eukaryontische Nukleotidsequenz aus dem Mikroorganismus isoliert wird. Hierbei findet pro übertragener eukaryontischer Nukleotidsequenz nur ein Transpositionsereignis statt. Bevorzugt wird die Übertragung von eukaryontischer cDNA.

In einer weiteren Variante des erfindungsgemäßen Verfahrens wird die sequenzunabhängige Insertionsmutagenese durch die Ausbildung eines Kointegrates während der Konjugation zwischen zwei Mikroorganismen erreicht.
Hierbei wird die eukaryontische Nukleotidsequenz in einen Mikroorganismus übertragen, der im weiteren als Donorzelle fungiert, anschließend wird die eukaryontische Nukleotidsequenz in dem Mikroorganismus durch sequenzunabhängige Insertionsmutagenese mutiert, wobei zunächst ein Kointegrat ausgebildet wird, dann das gebildete Kointegrat über Konjugation in einen anderen Mikroorganismus (Rezipient) übertragen wird, dort das Kointegrat aufgelöst wird, wobei die eukaryontische Nukleotidsequenz durch die Insertion einer zellfremden Nukleotidsequenz genetisch verändert wird und diese genetisch veränderte eukaryontische Nukleotidsequenz aus dem Mikroorganismus isoliert wird.
Unter zellfremder DNA ist erfindungsgemäß DNA zu verstehen, die natürlicher Weise nicht in Eukaryonten vorkommt, d.h. aus Prokaryonten oder ggf. aus Viren oder Phagen stammt und in Mikroorganismen repliziert wird. In einer bevorzugten Variante des erfindungsgemäßen Verfahrens werden cDNA-Sequenzen aus Eukaryonten, bevorzugt Pflanzen, Algen und/oder Pilzen, eingesetzt.

Wesentlich für das vorliegende Verfahren ist, daß die Mutagenese in dem Mikroorganismus mit hoher Effizienz, bevorzugt mit einer relativen Frequenz nach der Selektion in einem Bereich von etwa 90 bis 100%, besonders bevorzugt von größer 90 bis 99%, insbesondere von 99,9% erfolgt. Erfindungsgemäß findet dabei pro Nukleotidsequenz nur ein Insertionsereignis statt.

Da die Mutagenese der eukaryontischen Nukleotidsequenz erfindungsgemäß sequenzunabhängig, also zufällig, durch insertion einer zellfremden Nukleotidsequenz erfolgt, zeichnet sich das erfindungsgemäße Verfahren in vorteilhafter Weise dadurch aus, daß die eukaryontische Nukleotidsequenz mutiert werden kann, ohne ihre genaue Nukleotidsequenz oder bestimmte Restriktionsschnittstellen zu kennen.
Erfindungsgemäß werden, bezogen auf einen ausgewählten Bereich einer eukaryontischen Nukleotidsequenz, durch die sequenzunabhängige Mutagenese eine Vielzahl verschiedenartiger (chimärer) eukaryontischer Nukleotidsequenzen erzeugt, die sich lediglich durch den Insertionsort der zellfremden Nukleotidsequenz in die eukaryontische DNA voneinander unterscheiden.

Ferner eignet sich das erfindungsgemäße Verfahren auch zur Mutagenese einer sehr großen Anzahl verschiedener eukaryontischer Nukleotidsequenzen, wie sie beispielsweise in Form einer eukaryontischen Gen-Bank vorliegen. Ein wesentlicher Vorteil des erfindungsgemäßen Verfahrens ist dabei, daß beispielsweise eine komplette Gen-Bank in einem einzigen Mutagenese-Ansatz genetisch verändert werden kann. Dabei kann eine eukaryontische Gen-Bank sowohl aus genomischen Nukleotidsequenzen als auch ausgehend von cDNA verwendet werden, wobei der Einsatz von cDNA bevorzugt wird. Prinzipiell ist der Einsatz von natürlichen eukaryontischen Nukleotidsequenzen als auch von chemisch synthetisierten Nukleotidsequenzen möglich. Die chemische Synthese kann dabei ausgehend von Transkritptionsprodukten, in der Regel Gesamt-RNA oder Poly-(A)⁺-RNA, oder auch beispielsweise anhand von Proteinsequenzen unter Einbeziehung des Kodon-Gebrauchs der eukaryontischen Zellen erfolgen.

Erfindungsgemäß wird die eukaryontische Gen-Bank in einem einzigen Mutagenese-Ansatz mit einer statistischen Wahrscheinlichkeit im Bereich von 90-100%, bevorzugt 90-99%, besonders bevorzugt 99,9%, homogen mutagenisiert.

Da das Prinzip des erfindungsgemäßen Verfahrens auf einer sequenzunabhängigen Mutagenese beruht, ist die Herkunft der eukaryontischen DNA von untergeordneter Rolle. Bei der eingesetzten eukaryontischen DNA kann es sich um Nukleotidsequenzen aus Pflanzen, Algen und/oder Pilzen handeln. Erfindungsgemäß wird eine Nukleotidsequenz aus Niederen oder Höheren Pflanzen eingesetzt. Bevorzugt wird eine Nukleotidsequenz aus Niederen Pflanzen der Gattung Physcomitrella, Funaria, Ceratodon oder Dicranum eingesetzt. Besonders bevorzugt wird eine Nukleotidsequenz aus Physcomitrella patens eingesetzt. Bei den Pilzen kann es sich um Hefen und/oder filamentöse Pilze, bevorzugt phytopathogene Pilze, besonders bevorzugt der Gattung Fusarium handeln. Denkbar sind auch andere eukaryontische Systeme und daraus isolierte DNA. Die vorliegende Erfindung wird durch die zuvor gemachten Angaben zur Herkunft der DNA, die mutagenisiert werden soll, nicht eingeschränkt.

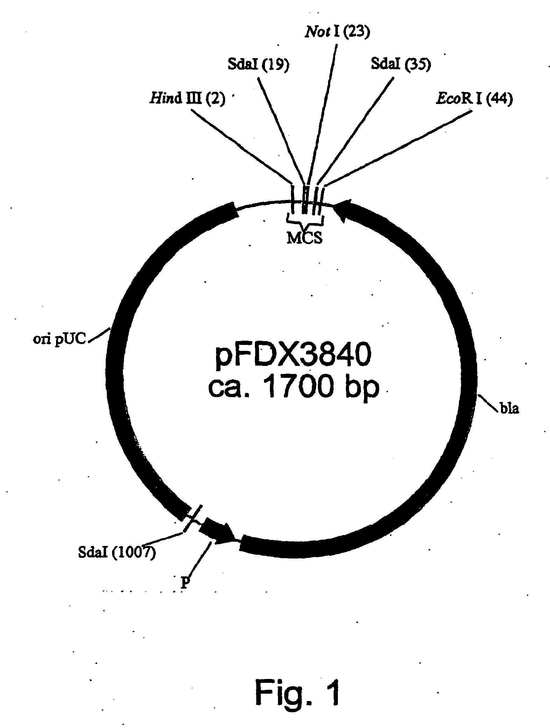

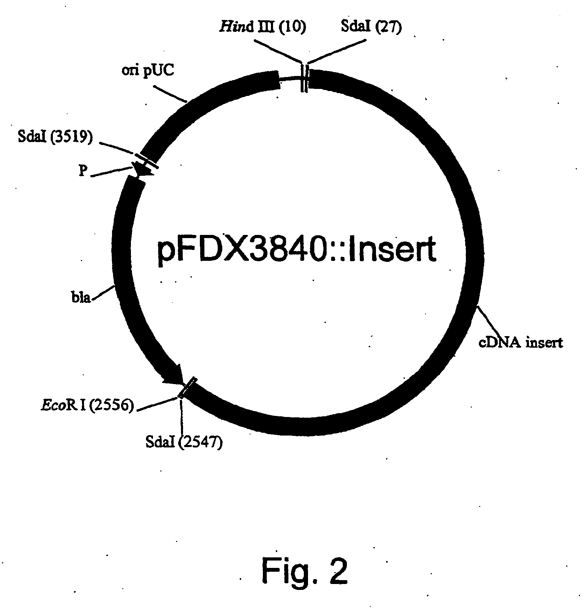

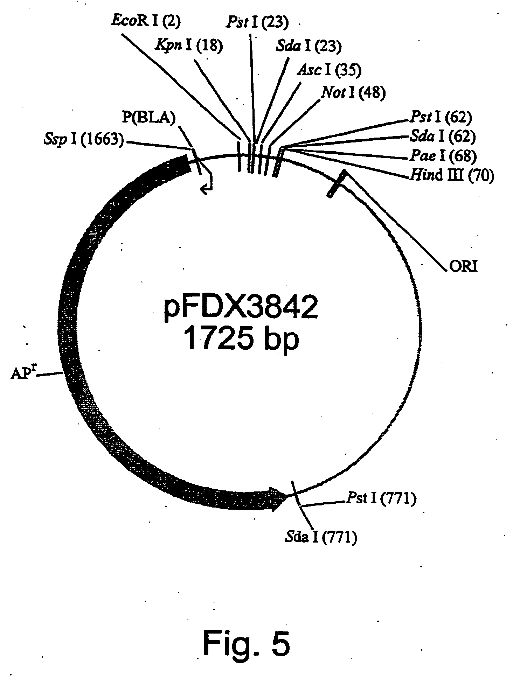

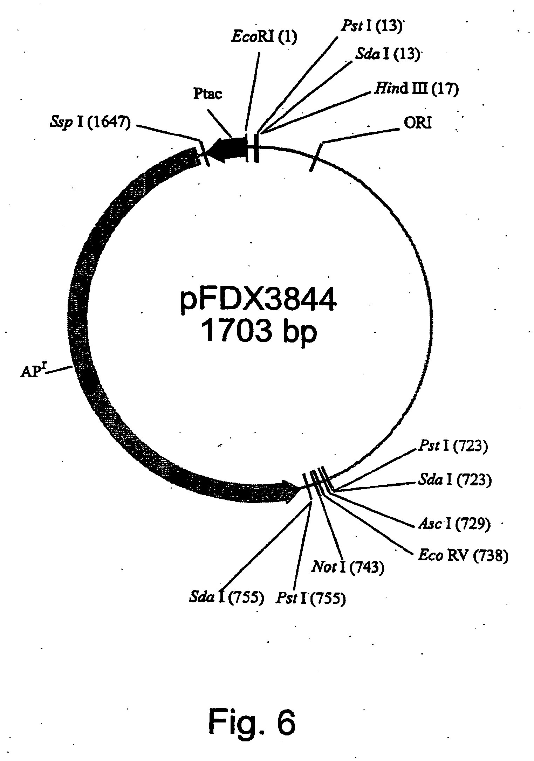

Erfindungsgemäß wird die eukaryontische Nukleotidsequenz nach an sich bekannten Methoden in einen geeigneten Mikroorganismus transferiert. Dazu kann die verwendete eukaryontische Nukleotidsequenz in einen entsprechend geeigneten Vektor, beispielsweise gemäß Fig. 1, eingebracht werden. Bevorzugt ist ein Vektor enthaltend natürliche Nukleotidsequenzen isoliert aus eukaryontischen Zellen und/oder anhand eukaryontischer DNA oder gemäß des Kodongebrauchs der eukaryontischen Zelle chemisch synthetisierte Nukleotidsequenzen. In weiteren Ausführungsvarianten der vorliegenden Erfindung wird ein Vektor enthaltend eine eukaryontische Nukleotidsequenz und zusätzliche funktionelle Nukleotidsequenzen, beispielsweise gemäß Figur 2 in einen Mikroorganismus übertragen. Unter zusätzlichen funktionellen Nukleotidsequenzen sind erfindungsgemäß u.a. beispielsweise ein Replikationsursprung zur Vermehrung in Bakterien, ein Selektionsmarker sowie zur Klonierung der eukaryontischen DNA geeignete Erkennungsstellen für Restriktionsendonukleasen zu verstehen. Beispiele für solche Konstruktionen weisen die Vektoren gemäß Fig. 1, 2, 5 oder 6 auf. Gegenstand der Erfindung sind somit auch Vektoren zum Einsatz in ein Verfahren der zuvor genannten Art mit Eigenschaften dargestellt in Fig. 1, 2, 5 oder 6. Hierbei stellen die Vektoren gemäß den Figuren 5 oder 6 Abkömmlinge von dem Vektor dargestellt in Fig. 1 dar.

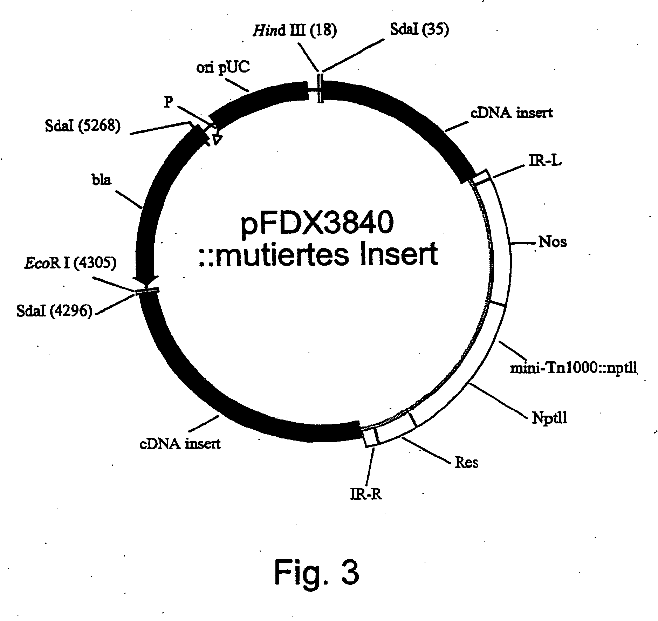

Ebenfalls offenbart ist auch die aus dem erfindungsgemäßen Verfahren resultierende genetisch veränderte eukaryontische DNA und/oder eine Population resultierender genetisch veränderter DNA-Sequenzen. Gegenstand der Erfindung ist ferner ein Vektor enthaltend genetisch veränderte eukaryontische DNA hergestellt gemäß dem vorliegenden Verfahren. Bevorzugt wird ein Vektor mit Eigenschaften gemäß Fig. 3. Darüber hinaus betrifft die vorliegende Erfindung auch einen genetisch veränderten Mikroorganismus, der eine genetisch veränderte eukaryontische DNA der zuvor beschriebenen Art oder einen Vektor enthaltend eine solche genetisch veränderte eukaryontische DNA enthält.

Wesentlich für das erfindungsgemäße Verfahren ist, daß die Mutagenese der eukaryontischen Nukleo6dsequenz in einem Mikroorganismus durch die Insertion einer zellfremden Nukleotidsequenz, bevorzugt prokaryontischen Ursprungs, erfolgt. Besonders bevorzugt handelt es sich erfindungsgemäß bei der inserierten prokaryontischen Nukleotidsequenz um eine Nukleotidsequenz, die Eigenschaften zur Transposition aufweist. In einer Ausführungsvariante der vorliegenden Erfindung wird ein Mini-Transposon verwendet, das erfindungsgemäß auf die wesentlichen Merkmale, die zur Transposition notwendig sind, reduziert ist. Insbesondere handelt es sich dabei um das sogenannte Mini-Transposon Mini-Tn1000::nptll.

Ein weiterer Vorteil des erfindungsgemäßen Verfahrens ist, daß die zur Insertion in die eukaryontische Nukleotidsequenz verwendete prokaryontische Nukleotidsequenz nur in Prokaryonten zur Transposition befähigt ist, nicht aber in Eukaryonten. Folglich zeichnet sich die durch das erfindungsgemäße Verfahren erzeugte Mutation in der eukaryontischen Nukleotidsequenz dadurch aus, daß sie im wesentlichen stabil ist.

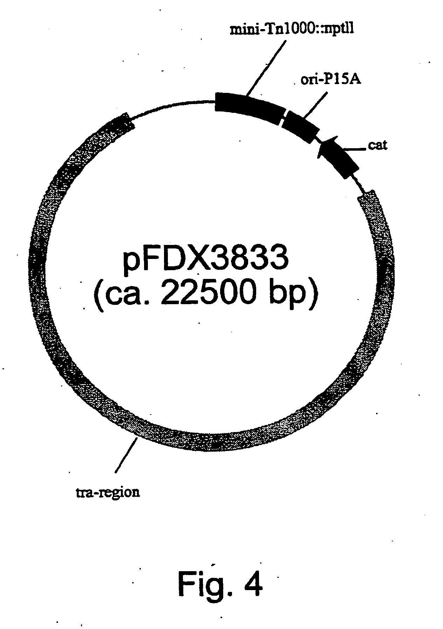

Erfindungsgemäß werden in dem zuvor beschriebenen Verfahren Mikroorganismen eingesetzt, welche die notwendigen Eigenschaften zur Transposition und Konjugation aufweisen. Der erfindungsgemäß verwendete Mikroorganismus enthält in replizierbarer Form ein Transposase-Gen und/oder ein Resolvase-Gen. Bevorzugt zeichnet sich der erfindungsgemäß eingesetzte Mikroorganismus dadurch aus, daß das Transposase-Gen und/oder das Resolvase-Gen unter der Kontrolle eines induzierbaren Promotors steht. Bevorzugt sind hier beispielsweise IPTGinduzierbare Promotoren, Arabinose-induzierbare Promotoren, wie beispielsweise der araBAD-Promotor oder Temperatur-induzierbare Promotoren, wie z.B. die Promotoren PL oder PR des Phagen λ, die ihrerseits durch den thermosensitiven Repressor c1857 kontrolliert werden. Die zur Transposition und Konjugation erforderlichen Gene können auch transient in dem Mikroorganismus vorliegen, d. h. zunächst verborgen vorliegen und nur unter bestimmten Umständen aktiviert werden.
Die zuvor erläuterten Eigenschaften zur Transposition und Konjugation können in dem erfindungsgemäß verwendeten Mikroorganismus chromosomal kodiert sein und/oder durch Vektoren vermittelt werden. Erfindungsgemäß enthält der genetisch veränderte Mikroorganismus insbesondere einen Vektor gemäß Fig. 4. Dieser Vektor vermittelt die notwendigen Eigenschaften zur Konjugation zwischen einer Donor- und Rezipientenzelle sowie der Übertragung von Nukleotidsequenzen. Außerdem enthält dieser Vektor eine prokaryontische Nukleotidsequenz, die zur Transposition geeignet ist. Bevorzugt wird in dem erfindungsgemäßen Verfahren als Mikroorganismus ein Bakterium der Gattung Enterobacteriaceae oder Bacillaceae eingesetzt. Besonders bevorzugt wird das Bakterium Escherichia coli verwendet und insbesondere die nicht-pathogene Zellinie E. coli K12.

Gegenstand der vorliegenden Erfindung ist weiter ein Verfahren zur Herstellung genetisch veränderter eukaryontischer Zellen der Gattung Physcomitrella oder deren Nachkommen, wobei in eine dieser zuvor genannten eukaryontischen Wirtszellen eine genetisch veränderte eukaryontische Nukleotidsequenz übertragen wird (welche zuvor gemäß dem erfindungsgemäßen Verfahren hergestellt wurde), diese transformierte eukaryontische Wirtszelle unter Bedingungen inkubiert wird, die eine gezielte homologe Rekombination der einbrachten genetisch veränderten eukaryontischen Nukleotidsequenz in das Genom der Wirtszelle ermöglichen und/oder auslösen, anschließend diejenigen eukaryontischen Wirtszellen identifiziert werden, die eine genetisch veränderte eukaryontische Nukleotidsequenz der zuvor genannten Art in ihrem Genom integriert enthalten und aus diesen Zellen entsprechendes Gewebe von Pflanzen, und/oder ganze Pflanzen regeneriert werden.
Unter den "Bedingungen", die in der erfindungsgemäß transformierten eukaryontischen Zelle eine gezielte homologe Rekombination ermöglichen und/oder auslösen, sind im Sinne der Erfindung beispielsweise die Inkubation der Zellen in Selektionsmedium oder eine Veränderung Kultivierungstemperatur zu verstehen. In einer Ausführungsvarinate der Erfindung kann beispielsweise eine Kultivierung in Kanamycin- und/oder G418-haltigem Kulturmedium und/oder eine Erhöhung der Kultivierungstemperatur von beispielsweise 37°C auf 42°C die erfindungsgemäße Insertionsmutagenese einer eukaryontischen DNA ermöglichen und/oder auslösen.

Darüber hinaus erfolgt die homologe Rekombination in diesem erfindungsgemäßen Verfahren mit einer hohen Effizienz, die allerdings von der Länge des eingesetzten eukaryontischen DNA-Fragments abhängig ist. Bevorzugt erfolgt die homologe Rekombination mit einer relativen Frequenz nach der Selektion in einem Bereich von etwa 0,1 bis 99,9 %, besonders bevorzugt von 1 bis 90 %, insbesondere von größer als 10 %.

Erfindungsgemäß wird bei diesem Verfahren eine genetisch veränderte eukaryontische Nukleotidsequenz oder ein Vektor enthaltend eine genetisch veränderte eukaryontische Nukleotidsequenz eingesetzt, die auf die weiter oben beschriebene Weise ebenfalls erfindungsgemäß hergestellt wird.

Wie bereits zuvor beschrieben, entsteht durch das erfindungsgemäße Verfahren eine Vielzahl genetisch veränderter, chimärer Nukleotidsequenzen, die sich lediglich durch den Insertionsort der zellfremden Nukleotidsequenz voneinander unterscheiden. Diese Vielzahl genetisch veränderter eukaryontischer Nukleotidsequenzen wird erfindungsgemäß über homologe Rekombination zur Herstellung einer Vielzahl genetisch veränderter eukaryontischer Organismen, d.h. Pflanzen, Algen und/oder Pilze genutzt. D.h. mit dem erfindungsgemäßen Verfahren wird eine Population eukaryontischer Zellen enthaltend verschiedenartig genetisch veränderte Nukleotidsequenzen erzeugt. Die einzelnen eukaryontischen Zellen zeichnen sich dabei dadurch aus, daß sie innerhalb eines engen genetischen Bereichs mutiert sind, sich aber alle durch den exakten Ort der Mutation voneinander unterscheiden. Das erfindungsgemäße Verfahren weist als weiteren Vorteil auf, daß eine Population genetisch veränderter eukaryontischer Zellen erzeugt wird, deren Genom mit einer statistischen Wahrscheinlichkeit im Bereich von 90-100%, bevorzugt von größer 90-99%, besonders bevorzugt von 99,9% an unterschiedlichen Stellen eine Veränderung trägt und sich somit zur Erstellung einer eukaryontischen Mutanten-Genbank eignet.

Dieses erfindungsgemäße Verfahren zeichnet sich ferner dadurch aus, daß die homologe Rekombination der genetisch veränderten eukaryontischen Nukleotidsequenz in das Genom der eukaryontischen Wirtszelle zu einer phänotypisch sichtbar und/oder meßbar veränderten Erscheinungsform in der Pflanze führt. D.h. in einer besonderen Ausführungsvariante der vorliegenden Erfindung kann eine eukaryontische Nukleotidsequenz zum Einsatz in das erfindungsgemäße Verfahren derart ausgewählt werden, daß die Insertionsmutagenese insbesondere innerhalb des kodierenden Bereichs eines aktiven Gens erfolgt. Die Funktionalität der mutierten Genorte kann bedingt durch den Insertionsort unterschiedlich stark verändert sein und erlaubt aufgrund dessen eine Feinkartierung verschiedener Domänen eines Gens. Erfindungsgemäß ist die resultierende genetisch veränderte eukaryontische Nukleotidsequenz spezifisch in einer Genfunktion zerstört, d.h. es werden Negativ-Mutanten erzeugt.

Der Integrationsort der zellfremden Nukleotidsequenz wird erfindungsgemäß durch gentechnische Methoden identifiziert. Als gentechnische Methoden sind hier alle an sich bekannten Methoden zu verstehen, mit denen die Integration bekannter Nukleotidsequenzen in die entsprechende Ziel-Sequenz identifiziert werden kann. Beispielhaft sei hier die Hybridisierung mit spezifischen und geeignet markierten Sonden genannt, die Identifizierung des Zielortes über Polymerasekettenreaktion (PCR) oder der Einsatz spezifischer Antikörper.

Das erfindungsgemäße Verfahren zeichnet sich ferner dadurch aus, daß als eukaryontische Wirtszellen Zellen der Gattung Physcomitrella eingesetzt werden. Besonders bevorzugt werden Zellen von Physcomitrella patens eingesetzt. Erfindungsgemäß werden auch genetisch veränderte Algen und/oder Pilze gemäß dem zuvor erläuterten Verfahren hergestellt. Bevorzugt werden genetisch veränderte filamentöse Pilze, besonders bevorzugt phytopathogene Pilze und insbesondere Pilze der Gattung Fusarium erzeugt.

Ein weiterer Vorteil des erfindungsgemäßen Verfahrens ist, daß die zur Insertion in die eukaryontische Nukleotidsequenz verwendete prokaryontische Nukleotidsequenz oder Teile davon nur in Prokaryonten zur Transposition befähigt ist, nicht aber in Eukaryonten. Folglich zeichnet sich die durch das erfindungsgemäße Verfahren erzeugte Mutation in der eukaryontischen Nukleotidsequenz dadurch aus, daß sie im wesentlichen stabil ist.

Gegenstand der vorliegenden Erfindung ist ferner eine genetisch veränderte eukaryontische Zelle der Gattung Physcomitrella oder deren Nachkommen hergestellt nach den zuvor genannten Verfahren. Dies umfaßt auch genetisch verändertes eukaryontisches Zell-Gewebe, fortpflanzungsfähiges Material, Samen und/oder Sporen von genetisch veränderten eukaryontischen Zellen Erfindungsgemäß umfaßt sind ferner ganze Pflanzen und/oder Pflanzenteile, die genetisch veränderte eukaryontische Zellen der zuvor beschriebenen Art enthalten und/oder aus solchen genetisch veränderten Zellen regeneriert werden. Besonders bevorzugt ist es eine Zelle von Physcomitrella patens.

Ferner zeichnet sich die erfindungsgemäß genetisch veränderte eukaryontische Zelle oder deren Nachkommen dadurch aus, daß sie phänotypisch sichtbar verändert ist und/oder eine eindeutig meßbar veränderte Erscheinungsform aufweist. Erfindungsgemäß trägt die genetisch veränderte eukaryontische Zelle und deren Nachkommen die integrierte zellfremde (prokaryontische) Nukleotidsequenz überwiegend in kodierenden Sequenzbereichen. Aufgrund dessen ist in den erfindungsgemäßen genetisch veränderten eukaryontischen Zellen und deren Nachkommen die Funktionalität der betroffenen Gene und der davon abgeleiteten Genprodukte in Abhängigkeit von dem Insertionsort der prokaryontischen Nukleotidsequenz unterschiedlich stark verändert. In einer besonderen Ausführungsvariante der vorliegenden Erfindung weisen die betroffenen Gene der genetisch veränderten eukaryontischen Zellen und deren Nachkommen keine Funktionalität mehr auf. Diese Mutationen werden auch als Negativ-Mutationen oder sogenannte "knock-out"- oder "loss of function"-Mutationen bezeichnet. Die entsprechend genetisch veränderten eukaryontischen Zellen und deren Nachkommen werden analog als Negativ- oder "Knock-out"-Mutanten bezeichnet.

Ferner zeichnen sich die erfindungsgemäßen genetisch veränderten eukaryontischen Zellen dadurch aus, daß sie durch sequenzunabhängige (zufällige) Insertion einer zellfremden Nukleotidsequenz genetisch stabil veränderte Nukleotidsequenzen enthalten. Bevorzugt enthält die erfindungsgemäß genetisch veränderte eukaryontische Zelle und deren Nachkommen eine stabil inserierte Nukleotidsequenz prokaryontischen Ursprungs. Dabei zeichnen sich die erfindungsgemäßen genetisch veränderten eukaryontischen Zellen und deren Nachkommen dadurch aus, daß sie eine in Prokaryonten und nicht in Eukaryonten transponierbare Nukleotidsequenz oder Teile davon enthalten.

Die vorliegende Erfindung betrifft außerdem die Verwendung eines Vektors gemäß Fig. 1, Fig. 5, Fig. 6 zur Klonierung und anschließenden Mutagenese einer eukaryontischen Nukleotidsequenz. Ferner sind auch die daraus resultierenden Vektoren enthaltend eine zur Mutation vorgesehene eukaryontische Nukleotidsequenz (im weiteren mit Abkömmlinge der Vektoren gemäß Fig. 5 oder Fig. 6 bezeichnet) offenbart. Die Erfindung betrifft ferner die Verwendung des Vektors gemäß Fig. 2 oder Abkömmlinge der Vektoren gemäß Fig. 5 oder Fig. 6 zur Herstellung einer genetisch veränderten eukaryontischen Nukleotidsequenz nach zuvor beschriebenem Verfahren.
offenbart ist weiter die Verwendung der zuvor genannten Vektoren, bevorzugt der Vektoren gemäß Fig. 2, Abkömmlinge der Fig. 5 oder Fig. 6 sowie ein Vektor gemäß Fig. 4 zum Einsatz in einen Mikroorganismus zur Mutagenese eukaryontischer Nukleotidsequenzen.

Ferner finden die genetisch veränderten eukaryontischen Zellen Einsatz zur Identifizierung funktioneller Nukleotidsequenzen in eukaryontischen Zellen und/oder zur Charakterisierung von eukaryontischen Nukleotidsequenzen unbekannter Funktion (funktionelle Genomanalyse).

Außerdem betrifft die vorliegende Erfindung die Verwendung genetisch veränderter eukaryontischer Zellen zum Einsatz in Bereichen der Landwirtschaft, der Pharmazie und/oder Medizin.

Im weiteren wird die vorliegende Erfindung durch Beispiele näher erläutert, die aber nicht limitierend für die Erfindung sind:

### 1) Allgemeine Klonierungsverfahren:

Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von Escherichia coli Zellen, Anzucht von Bakterien und Sequenzanalyse rekombinanter DNA wurden wie bei Sambrook et al. (1989) (Cold Spring Harbor Laboratory Press: ISBN 0-87969-309-6) beschrieben durchgeführt.

### 2) Chemikalien:

Die verwendeten Chemikalien wurden, sofern im Text nicht anders erwähnt, in Analyse-Qualität von den Firmen Fluka (Neu-Ulm), Merck (Darmstadt), Roth (Karlsruhe), Serva (Heidelberg) sowie Sigma (Deisenhofen) bezogen. Lösungen wurden mit aufbereitetem, pyrogenfreiem Wasser, im weiteren Text als H₂O bezeichnet, aus einer Milli-Q Water System Wasseraufbereitungsanlage (Millipore, Eschborn) angesetzt. Restriktionsendonucleasen, DNA-modifizierende Enzyme und molekularbiologische Kits wurden von den Firmen AGS (Heidelberg), Amersham (Braunschweig), Biometra (Göttingen), Boehringer (Mannheim), Genomed (Bad Oeynnhausen), New England Biolabs (Schwalbach/Taunus), Novagen (Madison, Wisconsin, USA), Perkin-Elmer (Weiterstadt), Pharmacia (Freiburg) Qiagen (Hilden) und Stratagene (Heidelberg) bezogen. Sie wurden, soweit nicht anders erwähnt, nach Herstellerangaben verwendet.

### 3) Pflanzenmaterial:

In einer Ausführungsvariante der vorliegenden Erfindung wurden Pflanzen der Art Physcomitrella patens (Hedw.) B.S.G. aus der Sammlung des Genetischen Arbeitsbereichs der Universität Hamburg verwendet. Sie gehen zurück auf den von H.L.K. Whitehouse in Gransden Wood, Huntingdonshire (England) gesammelten Stamm 16/14, der von Engel (1968, Am J Bot 55, 438-446) aus einer Spore subkultiviert wurde. Eine Vermehrung der Pflanzen erfolgte über Sporen und über Regeneration der Gametophyten. Aus der haploiden Spore entwickelt sich das Protonema als chloroplastenreiches Chloronema und chloroplastenarmes Caulonema, an dem sich nach etwa 12 Tagen Knospen bilden. Diese wachsen zu Antheriden und Archegonien tragenden Gametophoren aus. Nach Befruchtung entsteht der diploide Sporophyt mit einer kurzen Seta und der Sporenkapsel, in der die Meiosporen heranreifen.

### 4) Pflanzenanzucht:

Die Kultivierung erfolgte in einer Klimakammer bei 25 °C Lufttemperatur, 55 µmol s⁻¹ m⁻² Lichtstärke (Weißlicht; Philips TL 65W/25 Leuchtröhren) und einem Licht/Dunkelwechsel von 16/8 Stunden. Das Moos wurde entweder in Flüssigkultur mit Knop-Medium verändert nach Reski und Abel (1985, Planta 165, 354-358) oder auf KnopFestmedium mit 1% Oxoid-Agar (Unipath, Basingstoke, England) kultiviert.

Die zur RNA- und DNA-Isolierung verwendeten Protonemen wurden in belüfteten Flüssigkulturen kultiviert. Alle 9 Tage wurden die Protonemen zerkleinert und in frisches Kulturmedium überführt.

### 5) Plasmide:

### 5.1.) pFDX3840, pFDX3842 und pFDX3844:

Bei dem Plasmid pFDX3840 handelt es sich um einen minimalisierten Vektor zur Erstellung von eukaryontischen Genbanken, der möglichst wenig nicht-essentielle Zielsequenzen für die Integration des Mini-Transposons außerhalb des eukaryontischen DNA-Inserts enthält (Fig. 1). Ausgehend von dem käuflichen Plasmid pUC12, dessen Sequenz bekannt ist, wurde das bla-Gen, welches eine Ampicillin-Resistenz vermittelt sowie der Replikationsursprung (ori) via PCR nach an sich bekannten Methoden amplifiziert und mit einer multiplen Klonierungsstelle mit singulären Restriktionsschnittstellen (multiple cloning site, mcs) verknüpft. Diese multiple Klonierungsstelle enthält u.a. Schnittstellen für die Restriktionsenzyme in der Reihenfolge Kpnl, Pstl, Sdal, Notl, Sdal und EcoRl. Das Restriktionsenzym Sdal erkennt das Oktamer 5'CCTGCAGG3', welches in der Regel innerhalb von natürlich vorkommenden Nukleotidsequenzen selten schneidet (rare-cutter).
Die Plasmide pFDX3842 und pFDX3844 stellen Abkömmlinge des Plasmids pFDX3840 dar. Unter anderem sind die beiden letztgenannten Plasmide hinsichtlich ihrer multiple cloning site weiter optimiert, d.h. sie weisen weniger singuläre Erkennungsstellen für Restriktionsendonukleasen auf.

### 5.2.) pFDX3840::Insert:

Hierbei handelt es sich um ein pFDX3840-Derivat, bei dem in die Notl-Schnittstelle ein eukaryontisches DNA-Insert kloniert wurde (Fig.2). Das Plasmid pFDX3840 wird nach gängigen Methoden mit Notl verdaut, isoliert und mit DNA-Fragmenten eukaryontischen Ursprungs, die vorbereitet durch einen Restriktionsverdau entsprechend kompatible Enden aufweisen, ligiert. Aufgrund der zuvor beschriebenen selten schneidenden Sdal-Restriktionsschnittstelle innerhalb der multiplen Klonierungsstelle, die die eukaryontischen DNA-Inserts flankieren, können die eukaryontischen DNA-Inserts, die wie weiter unten beschrieben durch Insertionsmutagenese mutiert werden, durch einen Sdal-Restriktionsverdau unversehrt aus dem Vektor herausgeschnitten werden. Die Isolierung von Sdal-Restriktionsfragmenten eukaryontischen Ursprungs ist insofern vorteilhaft, als daß 4 bp lange hervorstehende 3'-Einzelstränge erzeugt werden, die bei der späteren homologen Rekombination in eukaryontischen Systemen die Effizienz der Rekombinationsereignisse positiv beeinflußt. Die Klonierung von eukaryontischer DNA kann in analoger Weise auch mit den Plasmiden pFDX3842 oder pFDX3844 erfolgen.

### 5.3.) pFDX3840::mutiertes Insert:

Hierbei handelt es sich um das unter 5.2.) beschriebene Plasmid, bei dem die eukaryontische DNA durch Transposonmutagenese und Insertion des Mini-Transposons Mini-Tn1000::nptll, wie nachfolgend beschrieben, mutiert wurde (Fig. 3).

### 5.4.) pFDX3832:

Ausgehend von dem konjugativen Plasmid R388 (Datta and Hedges, 1972, J. Gen. Microbiol., 72:349-355; Avila and de la Cruz, 1988, Plasmid, 20:155-157; EMBL-Accession-No: X81123), welches 33 kb groß ist und eine konjugative Transferregion (tra), einen Replikationsursprung zur vegetativen Vermehrung (ori), eine Sulfonamid-Resistenz (Sm) und eine Trimethoprim-Resistenz (Tp) trägt, werden die für eine Konjugation bedeutenden Teile subkloniert und große Bereiche des Vektors, die für die Funktion des Plasmids nicht bedeutend sind, insbesondere die Resistenzgene Sm und Tp verworfen. Das verkürzte Plasmid, welches die vollständigen Funktionen zur Konjugation, aber keine Resistenzgene mehr trägt, wurde dann mit einem Chloramphenicol-Resistenzgen (EcoRV-Sall-Fragment aus dem Plasmid pFDX900) und einem Replikationsursprung zur vegetativen Vermehrung (ori P15A) ausgestattet.

Konkret wurde der für die Konjugtion essentielle Vektoranteil aus dem Plasmid R388 als ein 19,5 kb langes Sall/Mscl-Fragment durch Restrkitionsverdau und anschließende Gelelektrophorese isoliert. Anschließend wurde analog ein 1947 bp langes Bspl-Klenow-Sall-Fragment aus dem Plasmid pFDX900 (Rak, B., 1988, Universität Freiburg; unveröffentlicht) isoliert, welches für eine Chloramphenicol-Resistenz (cat) kodiert und den vegetativen Replikationsursprung P15A trägt. Das Plasmid pFDX900 selbst ist ein pFDX733-Derivat, das statt einer Kanamycin-Resistenz (neo) eine Chloramphenicol-Resisitenz (cat) trägt. Dazu wurde ein 6994 bp langes Stul-BamHI-Klenow-Fragment von pFDX733 (Schnetz, et al., 1987, J. Bacteriol., 169:2579-2590) mit einem 964 bp langen Eco0109-Tthlll-Klenow-Fragment aus pBR325 ligiert.
Durch Ligation des 19,5 kb langes Sall/Mscl-Fragment (tra) aus R388 mit dem 1947 bp langen Bspl-Klenow-Sall-Fragment aus dem Plasmid pFDX900 (cat, ori), anschließende Transformation in einen E. coli Stamm und Selektion auf Chloramphenicol resistente Transformanten nach an sich bekannten Methoden wurde das Plasmid pFDX3832 erhalten.

### 5.5.) pFDX3809:

Das Plasmid pFDX3809, welches ein 1753 bp langes EcoRV-Fragment des Mini-Transposons (Mini-Tn1000::nptll) trägt, wird durch die nachfolgend beschriebenen Klonierungsschritte erhalten:
Die linke invertierte Sequenzwiederholung (IRL) wurde als synthetisches Oligonukleotid in die EcoRV-Schnittstelle des käuflichen Vektors pBluescript KS2 kloniert (resultierendes Plasmid pFDX3803). Anschließend wurde die rechte invertierte Sequenzwiederholung (IRR) zwischen die Pstl- und Xbal-Schnittstelle von pFDX3803 kloniert (resultierendes Plasmid pFDX3804). Die resolution site (res) wurde mit den unten aufgeführten Oligonukleotiden (Oligo 1, Oligo 2) ausgehend von dem Plasmid pFDX291 (pACYC177-Derivat, in das das Tn1000 ausgehend von einem F-Plasmid während einer Konjugation integrierte; Rak, B., Universität Freiburg; unveröffentlicht) amplifiziert und in die EcoRV-Schnittstelle des käuflichen Vektors pBluescript KS2 kloniert (resultierendes Plasmid pFDX3806). Anschließend wurde das Plasmid pFDX3804 und das Plasmid pFDX3806 in separaten Ansätzen mit den Restriktionsenzymen Hindlll/Pstl verdaut und zu dem resultierenden Plasmid pFDX3807 ligiert. Zur Einfügung einer multiplen Klonierungsstelle mit singulären Restriktionsschnittstellen (mcs; multiple cloning site) wurde das Plasmid pFDX3803 in einem Restriktionsansatz mit Avrl und Xbal verdaut. Ein entsprechendes Avrl/Xbal-Fragment wurde aus dem Plasmid pFDX3807 isoliert und mit dem zuvor beschriebenen pFDX3803-Restriktionsansatz zu dem Plasmid pFDX3808 ligiert, welches nunmehr ein Mini-Transposon trägt. In dieses Mini-Transposon wurde anschließend noch eine nptII-Genkassette unter der Kontrolle des nos-Promotors eingebaut. Diese nptII-Genkassette enthält vor dem nptII-Gen den nos-Promotor und hinter dem nptll-Gen den 3'-untranslatierten Bereich des nos-Terminators. Diese Genkassette wurde durch einen HindIII/BamHI-Klenow-Restriktionsverdau ausgehend von dem bakteriellen Transposon Tn5 aus dem Plasmid pBSNNN (Reski, R., Universität Freiburg) isoliert, wobei das nptll-Gen ursprünglich aus dem bakteriellen Transposon Tn5 stammt. Dieses Fragment wurde abschließend mit dem Plasmid pFDX3808, welches durch eine Mlul-KJenow-Behandlung liniearisiert wurde, ligiert. Das resultierende Konstrukt wird als pFDX3809 bezeichnet.

### Verwendete Primer:

IRL:
5'CCTAGGGCCGCTTAAGGTGCACGCGCACGCGTGCATGCAACGTAC
GTTTTCGTTCCATTGGCCCTCAAACCCCGTCGACGATATC3'

IRR:
5'AGAGCGCGATATCGGGGTTTGAGGGCCAATGGAACGAAAACGTAC
GTTCTG3'

Oligo 1: 5'CTGCAGCGTCCGAAATATTATAAATTATCGC3'
Olipo2: 5'CGATATTTGATTTAGGATACACCTAGG3'

### 5.6.) pFDX3833:

Das Plasmid pFDX3833 ist ein pFDX3832-Derivat und trägt zusätzlich ein 1753 bp großes EcoRV-Insert aus dem Plasmid pFDX3809 (Rak, B., Universität Freiburg; unveröffentlicht), welches ein Mini-Transposon (Mini-Tn1000::nptll) enthält. Das Mini-Tn1000 besteht aus den funktionellen Einheiten IRL-NOS-Promoter/nptll/NOS-Terminator-RS-IRR, wobei die Abkürzungen folgende Bedeutung haben: Tn = Transposon, IRL/IRR = invertierte Sequenzwiederholung links/rechts, nos = Nopalin-Synthase-Promotor aus Agrobacterium tumefaciens, nptll = Neomycin-Kanamycin-Phosphotransferase-Gen und RS = Auflösungsstelle (resolution site). Eine schematische Darstellung gibt Fig. 4 wieder.

### 6) Überprüfung der Funktionalität des konjugativen Plasmids pFDX3832:

Die Konjugation als Funktionsnachweis für das konjugative Plasmid wurde mit Hilfe des Donorstamms E. coli K12-R2117 (recA-Derivat des E. coli Wildtyps K12-W3110) durchgeführt. Da der Donorstamm das Plasmid pFDX3832 trägt, wächst er in Medium mit einer Konzentration von 10 µg/ml Chloramphenicol (Cm). Der Rezipient E. coli R1037 besitzt chromosomal ein Streptomycinresistenz-Gen (Strep) und wächst in Medium mit Streptomycin-Konzentrationen von 100 µg/ml.

Jeweils 20 ml LB-Medium (mit entsprechendem Antibiotikum) werden mit einer Über-Nachtkultur von Donor und Rezipient (OD₆₀₀-Start: 0,1) angeimpft. Nach Erreichen der OD₆₀₀ von 0,4 - 0,5 werden je 3 ml der Bakterienkultur von Donor und Rezipient gemischt und ohne Selektionsdruck bis zu einer OD₆₀₀ von 0,8 weiter kultiviert. Dann werden 0,3 ml der Mischung auf Medium ohne Selektionsdruck ausplattiert und 1,5 h bei 37°C inkubiert. In diesem Zeitraum findet die Konjugation statt, d.h. das Plasmid pFDX3832 wird in den Rezipientenstamm übertragen.

Anschließend werden die Zellen abgeschwemmt und in geeigneten Verdünnungen auf Selektiv-Medium mit den Antibiotika Streptomycin (zur Bestimmung des Rezipiententiters) bzw. Streptomycin und Chloramphenicol (zur Bestimmung des Titers an Exkonjuganten) ausplattiert. Die Donorzellen mit dem Plasmid pFDX3832 sind nur gegen Chloramphenicol resistent und die Rezipientenzellen nur gegen Streptomycin. Nur Rezipientenzellen, die via Konjugation das Plasmid pFDX3832 erhalten haben, sind gegen beide Antibiotika (Cm und Strep) resistent. Die Konjugationsrate berechnet sich aus der Anzahl an Exkonjuganten geteilt durch die Anzahl aller Rezipientenzellen nach erfolgter Konjugation. Die Konjugationsrate für pFDX3832 beträgt 57 - 86% gegenüber einer unter den zuvor beschriebenen Bedingungen ansonsten üblichen Konjugationsrate von 30 -40 %.

### 7) Herstellungneuer Donor- bzw. Rezipienten-Stämme von E. coli:

Für die Integration von beliebigen DNA-Fragmente in das Genom von *E. coli* durch ortsspezifische Rekombination an der attB-Integrationsstelle werden zwei Hilfssysteme benötigt: a) ein temperatursensitives Helferplasmid, b) ein Vektor mit λattP-Sequenzen und dem Resolvase- bzw. Transposase-Gen hinter einem induzierbaren Promotor.

### a) Temperatursensitives Helferplasmid pFDX3401

Das Plasmid trägt folgende Eigenschaften:
- ori = Replikationsursprung zur vegetativen Vermehrung
- cat = Chloramphenicol-Resistenz-Gen
- λcl₈₅₇^{ts} = ein temperatursensitiver Repressor
- λP_{R}, ein unter der Kontrolle des Repressors stehender Promotor
- int = Integrase-Gen
- rep^{ts} = temperatursensitive, vegetative DNA-Replikation

### b) Klonierungsvektor mit Polylinker und λattP-Sequenzen pFDX3801

Der Vektor trägt folgende Eigenschaften:
- ori = Replikationsursprung zur vegetativen Vermehrung
- kan= Kanamycin-Resistenz-Gen
Flankiert von zwei Notl-Schnittstellen liegen vor:
- λattP = Integrationsstelle
- Tet^{R} = Tetracyclin-Resistenz-Gen
- MCS = Multiple cloning site mit i)- oder ii)-lnsert
- i) lacl^{q}-IacOi-Ptac-lacO1-SD mit tnpR = IPTG-induzierbarer Promotor mit Resolvase-Gen
oder
- ii) lacl^{q}-lacOi-Ptac-lacO1-SD mit tnpA = IPTG-induzierbarer Promotor mit Transposase-Gen

Die Konstrukte werden mit drei verschiedenen Shine Dalgarno-Sequenzen (SD) kloniert, um die Resolvase und Transposase unterschiedlich stark exprimieren zu können und um so die optimalen Enzymkonzentrationen von Resolvase und Transposase finden zu können.

Aus den Konstrukten wurde jeweils das Notl-Fragment isoliert und religiert, so daß die religierten Moleküle keinen Replikationsursprung mehr tragen und zur Selektion das Tetracyclin-Resistenzgen enthalten.

Die drei Transposase-Konstrukte werden jeweils in den E. coli Donorstamm R2117, der das Helferplasmid pFDX3401 enthält, transformiert. Die drei Resolvase-Konstrukte werden jeweils in den E. coli Rezipientenstamm R1037, der ebenfalls das Helferplasmid pFDX3401 enthält, transformiert.
Die Bakterien werden anschließend auf LB-Medium mit Tetracyclin ausplattiert und bei einer Temperatur von 42°C inkubiert.
Dadurch wird der temperatursensitive Repressor inaktiviert, wodurch es zur Synthese der Integrase kommt. Diese katalysiert die ortsspezifische Rekombination zwischen der λattP-Stelle auf dem Notl-Fragment und der AattB-Stelle im Chromosom und damit die Integration der Expressionskassette in das Chromosom. Gleichzeitig wird das für die Replikation des Helferplasmids essentielle Rep-Protein (kodiert durch das rep^{ts}-Allel) inaktiviert, so daß die Replikation des Helferplasmids blockiert ist und dieses im weiteren Verlauf der Bakterienvermehrung verloren geht.

Somit werden bei 42°C Tetracyclin-resistente Kolonien selektioniert. Diese tragen die Resolvase- bzw. Transposase-Konstrukte in das Bakterienchromosom integriert. Aus Übernacht-Kulturen werden DMSO-Dauerkulturen angelegt.
Im folgenden sind die wesentlichen Eigenschaften der E. coli Donor- bzw. Rezipienten-Stämme noch einmal aufgelistet:

### E. coli R2117-Derivate (Donor)

- konjugatives Plasmid pFDX3833 mit Mini-Tn1000::nptII
- chromosomal integriert in λattB: lacl^{q}- OᵢtacOP - tnpA; tet^{R}
- drei verschiedene Shine Dalgarno Sequenzen aus
   a) Phagen T7-Gen10
   b) natürliche Sequenz
   c) E. coli bglG-Gen
- recA⁻
- strep^{S}

Die Abkürzungen bedeuten dabei:
lacl^{q} = Laktose Repressorgen mit Mutation im Promotorbereich
tnpA = Transposase-Gen

OᵢtacOP = tac-Operator-Promotor, wobei hier die Pribnow-Box die Konsensus-Sequenz TATAAT und die -35-Box die Konsensus-Sequenz TTGACA aufweist. Oᵢ bezeichnet die Tatsache, daß 92 bp stomaufwärts (5') des Operators ein zweiter synthetischer Operator mit idealen (Oᵢ) Bindeeigenschaften für den Repressor besitzt. Hierdurch kommt es zu einer kooperativen Bindung des Repressors an beide Promotoren (loop-Bildung) und somit zu einer wesentlich verbesserten Repression.

### E. coli R1037-Derivate (Rezipient)

- konjugatives Plasmid pFDX3833 mit Mini-Tn1000::nptll
- chromosomal integriert in λattB: lacl^{q}- OᵢtacOP - tnpR; tet^{R}
- drei verschiedene Shine Dalgarno Sequenzen aus
   d) Phagen T7-Gen10
   e) natürliche Sequenz
   f) E. coli bglG-Gen
- strep^{R}
Hierbei steht tnpR für Resolvase-Gen.

### 8) Transformation des Donorstammes mit eukaryontischer DNA:

Der E. coli Donorstamm wird nach an sich bekannten Methoden zur Übertragung von DNA kompetent gemacht. Anschließend werden 1 ng - 1 µg eukaryontischer DNA oder Vektor-DNA (enthaltend Ampicillin-Resistenzgen) enthaltend eukaryontische DNA in den Elektroporationsansatz eingesetzt. Bei Einsatz einer DNA-Bank wird die DNA-Menge nach den Erfahrungen des Fachmannes derart gewählt, daß gewährleistet ist, daß die DNA-Bank in ihrer Gesamtheit ein- bis zweimal vertreten ist. Die Selektion der erfolgreich transformierten Zellen erfolgt auf LB-Medium mit den Antibiotika Ampicilin (50µg/ml), Chloramphenicol (10 µg/ml) und Tetracyclin (10 µg/ml). Aus entsprechend kultivierten Über-Nachtkulturen werden DMSO-Dauerkulturen angelegt.

### 9) Konduktion:

Der Donorstamm wird in 20 ml LB-Medium mit Ampicilin (50µg/ml), Chloramphenicol (10 µg/ml) und Tetracyclin (10 µg/ml) in einer Verdünung von 1:100 aus der DMSO-Dauerkultur angeimpft. Im Anschluß daran wird mit IPTG (Isopropyl-β-D-thiogalaktopyranosid) die Expression des Transposase-Gens induziert.
Der E. coli Rezipientenstamm wird in 20 ml LB-Medium mit Streptomycin (100 µg/ml) und Tetracyclin (6 -10 µg/ml) in einer Verdünung von 1:100 aus der DMSO-Dauerkultur angeimpft. Auch hier wird in Anschluß daran mit IPTG die Expression der Resolvase induziert.
Beide Bakterienstämme werden bis zu einer OD₆₀₀ von 0,4 kultiviert. Dann wird IPTG (1 mM Endkonzentration) zugesetzt und jeweils bis zum Erreichen einer OD₆₀₀ von 0,8 weiter kultiviert. Anschließend werden je 150 µl von Donor und Rezipientenkultur gemischt und auf eine LB-Nähragarplatte ohne Antibiotika, aber enthaltend 1 mM IPTG, ausgestrichen. Nachdem die Ausstriche 10 min mit leicht geöffnetem Deckel getrocknet sind, werden die Agarplatten 1,5 h bei 37°C inkubiert.
Nach der Inkubation werden die Bakterien mit 3 ml LB-Medium ohne Antibiotika abgeschwemmt und auf Selektivmedium (LB-Medium mit Ampicillin, Streptomycin, Tetracyclin in Konzentrationen wie zuvor beschrieben) ausplattiert und bei 37° C über Nacht inkubiert.
Zur Kontrolle und zur Titerbestimmung werden geeignete Verdünnungen auf Nähragarplatten mit Streptomycin (zur Bestimmung des Titers aller Rezipienten), Streptomycin und Chloramphenicol (zur Bestimmung des Exkonjugantentiters) sowie Ampecillin und Streptomycin (zur Bestimmung des Titers an Exkonduktanten) ausplattiert. Die typische Rate an Exkonjuganten beträgt 50 - 80 % und die Rate an Exkonduktanten 6 - 8 %.
Die erhaltenen Exkonduktanten werden von den Agarplatten abgeschwemmt. Die abgeschwemmten Bakterien werden in 5 Liter Selektivmedium (LB-Medium mit Ampicillin und Streptomycin) inokuliert und über Nacht bei 37 °C angezüchtet. Aus diesem Ansatz werden DMSO-Dauerkulturen angelegt und aus der restlichen Kulturflüssigkeit die Plasmid-DNA quantitativ nach gängigen Methoden, wie beispielsweise CsCI-Gradient oder Affinitätschromatographie, isoliert. Die erhaltene DNA wird dann entsprechend in eukaryontische Zellen überführt.

### 10) PEG-vermittelter DNA-Transfer:

Die Transformation von Physcomitrella patens wurden mit dem Polyethylenglycol (PEG) vermittelten direkten DNA-Transfer in Protoplasten durchgeführt. Die Reinigung der zu transformierenden Plasmid-DNA erfolgte über Qiagen Tip-500 Säulen (Diagen, Hilden). Das PEG hatte das Molekulargewicht 4000 und wurde 3 Stunden vor der Transformation jeweils frisch in 3M-Medium (Schäfer et al., 1991, Mol Gen Genet 226, 418-424: 15 mM MgC12, 0,1 % MES, 0,48 M Mannit, pH 5,6, 580 mosmol) angesetzt. Alle verwendeten Lösungen waren sterilfiltriert.

Die Isolierung der Protoplasten erfolgte nach der Methode von Rother et al.(1994, J. Plant Physiol 143, 72-77) aus Flüssigkulturen. Nach dem letzten Waschschritt wurden die Protoplasten in 3M-Medium aufgenommen, gezählt, mit 3M-Medium auf eine Konzentration von 1,2x10⁶ Protoplasten/ml eingestellt und sofort zur Transformation eingesetzt.

Die Transformation erfolgte mit Abänderungen nach einer Methode von Schäfer et al. (1991, Mol Gen Genet 226, 418-424) in sterilen Glasröhrchen bei Raumtemperatur. In den Glasröhrchen wurden zunächst 50 µg Plasmid-DNA in 100 µl 0,1 M Ca(NO₃)₂ mit 250 µl Protoplasten gemischt, dann 350 µl 40 % (w/v) PEG 4000 tropfenweise unter vorsichtigem Umschwenken dazugegeben. Während der folgenden 30-minütigen Inkubation wurde der Transformationsansatz alle 5 Minuten vorsichtig geschwenkt. Anschließend folgte eine schrittweise Verdünnung durch Zugabe von je 1, 2, 3 und 4 ml 3M-Medium über 20 Minuten. Die Protoplasten wurden dann 5 Minuten bei 70xg sedimentiert, in 3 ml Regenerationsmedium (Rother et al. 1994, J. Plant Physiol 143, 72-77) aufgenommen und auf zwei Petrischalen mit einem Durchmesser von 3 cm verteilt. Die Kultivierung der Protoplasten erfolgte zunächst 24 Stunden bei einer geringeren Lichtstärke von 4,9 µmol s⁻¹ m⁻² und anschließend bei einer Lichtstärke von 46,8 µmol s⁻¹ m⁻² in einer Klimakammer bei 25 °C mit einem Licht/Dunkelwechsel von 16/8 Stunden.

Die nach 5-6 Tagen ausgekeimten Protonemen wurden auf Knop-Platten übertragen, die mit steriler Cellophanfolie (Rother et al., 1994, J Plant Physiol 143, 72-77) beschichtet waren. Nach weiteren 7 Tagen wurde die Cellophanfolie mit den nun bis zu 15-zelligen Protonemafäden auf Knop-Platten mit den Antibiotika G418 (50 mg/l) umgesetzt. Für die Selektion von stabilen Transformanden wurden am 26. Tag nach der Transformation die überlebenden Protonemen von den Knop-Platten isoliert und auf Knop-Platten ohne Cellophanfolie und ohne Antibiotikum umgesetzt. Nach einer weiteren Kultur von 14 Tagen wurde wieder auf Selektionsplatten umgesetzt. Am 54. Tag nach der Transformation wurden die überlebenden stabil transformierten Pflanzen in Flüssigkultur ohne Antibiotikum überführt.

### 11) Gesamt-DNA Isolierung aus Physcomitrella patens:

Die Angaben zur Isolierung von Gesamt-DNA beziehen sich auf die Aufarbeitung von einem Gramm Frischgewicht an Pflanzen material.

CTAB-Puffer: 2% (w/v) N-Cethyl-N,N,N-trimethylammoniumbromid (CTAB); 100 mM Tris-HCl pH 8,0; 1,4 M NaCl; 20 mM EDTA.

N-Laurylsarkosin-Puffer: 10 % (w/v) N-Laurylsarkosin; 100 mM Tris-HCl pH 8,0; 20 mM EDTA.

Das Pflanzenmaterial wurde in einem Mörser unter flüssigem Stickstoff zu einem feinen Pulver zerrieben und in 2 ml Eppendorfgefäße überführt.

Das gefrorene Pflanzenmaterial wurde anschließend mit 1 ml Aufschlußpuffer (1ml CTAB-Puffer, 100 ml N-Laurylsarkosin-Puffer, 20 ml β-Mercaptoethanol und 10 ml Proteinase K-Lösung, 10 mg/ml) überschichtet und unter ständigem Schütteln eine Stunde bei 60 °C inkubiert. Das erhaltene Homogenat wurde auf zwei Eppendorfgefäße (2ml) aufgeteilt und zweimal mit dem gleichen Volumen Chloroform/Isoamylalkohol (24:1) ausgeschüttelt. Zur Phasentrennung wurde jeweils 15 min bei 8.000x g und Raumtemperatur zentrifugiert. Anschließend wurde die DNA mit eiskaltem Isopropanol bei -70°C 30 min gefällt. Die gefällte DNA wurde bei 4 °C und 10.000 g für 30 min sedimentiert und in 180 ml TE-Puffer (Sambrook et al., 1989, Cold Spring Harbor Laboratory Press: ISBN 0-87969-309-6) resuspendiert. Zur weiteren Reinigung wurde die DNA mit NaCl (1,2 M Endkonzentration) versetzt und mit dem zweifachen Volumen absoluten Ethanol 30 min bei - 70 °C erneut gefällt. Nach einem Waschschritt mit 70% Ethanol wurde die DNA getrocknet und im Anschluß in 50 ml H₂0 mit RNAse (50 mg/ml Endkonzentration) aufgenommen. Die DNA wurde über Nacht bei 4 °C gelöst und nachfolgend der RNAse-Verdau 1h bei 37 °C durchgeführt. Die Lagerung der DNA erfolgte bei 4°C.

### 12) Isolierung von Gesamt-RNA und poly-(A)⁺ RNA aus Physcomitrella patens:

Für die Untersuchung von Transkripten und zur Herstellung von cDNA wurde sowohl Gesamt-RNA als auch poly-(A)⁺ RNA isoliert. Die Gesamt-RNA wurde mit dem RNeasy Plant Total RNA Kit (Qiagen, Hilden) und dem darin enthaltenen RLT-Puffer nach Angaben des Herstellers gewonnen. Aus der gewonnenen Gesamt-RNA wurde die poly-(A)+ RNA mit dem Poly AtractmRNA Isolation System III der Firma Promega (Heidelberg) nach Angaben des Herstellers isoliert.

Nach Konzentrationsbestimmung der RNA bzw. der poly (A)+ RNA wurde die RNA durch Zugabe 1/10 Volumen 3 M Natriumacetat pH 4,6 und 2 Volumen Ehanol gefällt und bei -70 °C gelagert.

### 13) Herstellung von cDNA anhand von Gesamt-RNA oder poly-(A)⁺ RNA aus Physcomitrella patens

Die Herstellung der cDNA erfolgte mittels eines cDNA-Synthesekit der Firma Boehringer Mannheim, Deutschland nach Herstellerangaben. Dabei wurden 5 µg poly-(A)⁺ RNA aus Physcomitrella patens eingesetzt. Die Erstellung der cDNA-Genbank erfolgte in dem Phagen λ-GT10 nach gängigen molekularbiologischen Methoden, wie bei Sambrock et al., 1989, Cold Spring Harbor Laboratory Press: ISBN 0-87969-309-6 beschrieben.

### 14) Northem-Hybridisierung:

Für die RNA-Hybridisierung wurden 20 mg Gesamt-RNA bzw. 1 mg poly-(A)⁺ RNA in 1,25%igen Agarosegelen mit Formaldehyd gelelektrophoretisch wie bei Amasino beschrieben aufgetrennt (1986, Anal. Biochem. 152, 304), kapillar mit 10x SSC auf positiv geladene Nylonmembranen (Hybond N+, Amersham, Braunschweig) übertragen, durch UV-Licht immobilisiert und für drei Stunden bei 68 °C mit Hybridisierungspuffer (10% Dextransulfat w/v, 1 M NaCl, 1% SDS, 100 mg Heringsperma-DNA) prähybridisiert. Die Markierung der DNA-Sonde mit dem 'Rediprime DNA-Labelling-Kit' (Amersham, Braunschweig) mit alpha-12P DCTP (Amersham, Braunschweig) während der Prähybridisierung durchgeführt. Die Hybridisierung erfolgte nach Zugabe der markierten DNA-Sonde in dem selben Puffer bei 68 °C über Nacht. Die Waschschritte erfolgten zweimal für 15 min mit 2x SSC und zweimal für 30 min mit 1x SSC, 1 % SDS bei 68 °C. Die Exposition der eingeschweißten Filter erfolgte bei -70 °C für die Dauer von 1-14 Tagen.

### 15) Herstellung einer strangspezifischen DNA-Sonde zur Hybridisierung mit Tn1000::nptII-Sequenzen:

Die Herstellung von Sonden erfolgt nach an sich bekannten Methoden. Die Markierung kann radioaktiv oder nicht-radioaktiv erfolgen und ist ebenfalls gängig.

### 16) PCR mit regeneriertem Pflanzenmaterial:

Um die putativen Transformanten der Pflanzen schon zu einem frühen Zeitpunkt auf den Besitz transformierter DNA zu testen, wurde die PCR eingesetzt. Zur Überprüfung wurden 0,01-0,05 mg Pflanzenmaterial unter sterilen Bedingungen den Kulturschalen entnommen, die Proben umgehend in flüssigem Stickstoff eingefroren und bis zur PCR maximal 24 h bei -70 °C gelagert. Auf das gefrorene Pflanzenmaterial wurde 50 µl des unten aufgeführten PCR-Mix gegeben und gemäß Temperaturprotokoll (siehe unten) die PCR durchgeführt. Mit dem Primerpaar PT1/PT2 wird ein 700 bp großer Teil des npt II Gens amplifiziert, sofern die Pflanze die zur Transformation verwendete DNA enthält.
Primer PT1: GAGGCTATTCGGCTATGACTG
Primer PT2: ATCGGGAGCGGCGATACCGTA

### PCR-Mix:

41 µl H₂O; 5 µl 10x PCR-Puffer mit MgCl₂ (AGS), 0,5 µl dNTP Mix (Pharmacia; 200 mM), 1,5 µl Primer PT1 (15 pmol); 1,5 µl Primer PT2 (15 pmol); 0,5 µl Taq-DNA-Polymerase (2U).

**Temperatur-Protokoll:**

| | |
|---|---|
| 1. | 95°C 1 min |
| 2. | 60°C 1 min |
| 3. | 72°C 2 min |
| 4. | 95°C 45 s |
| 5. | 60°C 1 min |
| 6. | 72°C 2 min (40 Zyklen Schritt 4.-6.) |
| 7. | 4°C bis zur Weiterverarbeitung |

### 17) PCR mit isolierter genomischer DNA:

Die beschriebene PCR wurde ebenfalls für den Nachweis des npt II Gens aus genomischer DNA verwendet. Als "Template"-DNA wurden 500 ng eingesetzt. Zur Analyse einer homologen Rekombination bei der Transformation mit dem Gendisruptionskonstrukt für das ftsZ-Gen wurden PCRs mit den Primerpaaren Ppf4/RT1 und RT4/Ppf5 und 500 ng Gesamt-DNA durchgeführt. Der Reaktions-Mix enthält auch hier die unter Punkt 16) der Beschreibung aufgeführten Komponenten.
Primer Ppf4: GGAGCTGACATGGTTTTCGT
Primer RT1: TGTCGTGCTCCACCATGTTG
Primer RT4:GTTGAGCATATAAGAAACCC
Primer Ppf5: AACCCATACTTAACTAGGCA

Es wurde folgendes Temperatur-Protokoll verwendet:

| | |
|---|---|
| 1. | 95°C 1 min |
| 2. | 55°C 1 min |
| 3. | 72°C 2 min 30 s |
| 4. | 95°C 45 s |
| 5. | 55°C 1 min |
| 6. | 72°C 2 min 30 s |
| 7. | 95°C 45s |
| 8. | 55°C Imin |
| 9. | 72°C 5 min (40 Zyklen Schritt 7.-9.) |
| 10. | 4°C bis zur Weiterverabeitung |

### 18) DNA-Sequenzierung:

Die Sequenzierungen erfolgten nach Sanger et al. (1977 Proc. Natl. Acad. Sci. USA 74, 5463-5467) mit dem ABI PRISMTM Dye Terminator Cycle Sequencing Core Kit (Perkin Elmer, Weiterstadt) und einem automatischen Sequenzierer vom Typ 373A der Firma Applied Biosystems (Weiterstadt). An Stelle der empfohlenen "Template-und Primermenge wurde 1,5 mg Template-DNA und 15 pmol Primer eingesetzt. Zur Sequenzierung von PCR-Amplifikaten wurde der PCR-Reaktionsansatz mit dem Nucleotrap PCR Extraction Kit von Macherey und Nagel (Düren) aufgereinigt und mit den zur Amplifikation eingesetzten Primern sequenziert.

### 19) Elektronenmikroskopie:

Moosprotonemen aus Flüssigkultur wurden für drei Stunden in 2,5% Glutaraldehyd/ 0,05 M Phosphatpuffer pH 7 bei 4 °C fixiert (Geyer, G. 1973 in: Ultrahistochemie, Gustav Fischer Verlag Stuttgart). Nach dreimaligem Waschen in Phosphatpuffer wurden die Proben bei 4 °C über Nacht in 1% (w/v) Osmiumtetroxid (OS04)/0,1 M Cadodylatpuffer pH 7,2 (Geyer, G. 1973 in: Ultrahistochemie, Gustav Fischer Verlag Stuttgart) inkubiert, dann mehrmals mit Cadodylatpuffer gewaschen, in 1% (w/v) Agar überführt und in einer aufsteigenden Alkoholreihe (15-100%) entwässert. Im Anschluß erfolgte die Einbettung in Spurr-Medium (Spurr, A.R. 1969, J. Ultrastr. Res. 26, 31-43).

Mit Hilfe eines Ultramikrotoms IomU2, (Reichert-Jung, Leica, Nußloch, Deutschland) wurden Ultradünnschnitte der Proben hergestellt und auf mit Mowital (Hoechst) befilmte Kupfernetzte übertragen. Die Schnitte wurden für 15-20 min mit einer gesättigten Uranylacetatlösung in 70% Methanol (Schreil, W. 1964, J. Cell Biol. 22, 1-20) nachkontrastiert und anschließend für 10-15 min mit Bleicitrat (Reynolds, E.S. 1963, Cell Biol. 17, 208-21) inkubiert. Die Auswertung erfolgte im Elektronenmikroskop bei einer Beschleunigungsspannung von 80 kV.

Legende zu den Figuren:
Fig.1: Schematische Darstellung des Vektors pFDX3840 geeignet zur Klonierung eukaryontischer Nukleotidsequenzen und Übertragung in Mikroorganismen
Fig. 2: Schematische Darstellung des Vektors pFDX3840::Insert enthaltend zusätzlich eukaryontische Nukleotidsequenzen geeignet zur Übertragung in Mikroorgansimen zwecks anschließender TransposonMutagenese.
Fig.3: Schematische Darstellung des Vektors pFDX3840::mutiertes-Insert enthaltend durch Transposonintegration (Mini-Tn1000::nptll) genetisch veränderte eukaryontische Nukleotidsequenzen (cDNA-Insert), welche ihrerseits zur homologen Rekombination in eukaryontischen Zellen geeignet sind.
Fig. 4: Schematische Darstellung des konjugativen Plasmids pFDX3833, welches das Mini-Transposon Tn1000::nptll enthält
Fig. 5: Schematische Darstellung des Vektors pFDX3842, einem Abkömmling des Vektors pFDX3840, welcher ebenfalls zur Klonierung eukaryontischer Nukleotidsequenzen und Übertragung in Mikroorganismen geeignet ist. Er unterscheidet sich von dem Vektor pFDX3840 durch einen anderen Polylinker zur Klonierung von eukaryontischer DNA.
Fig. 6: Schematische Darstellung des Vektors pFDX3844, einem Abkömmling des Vektors pFDX3840, welcher ebenfalls zur Klonierung eukaryontischer Nukleotidsequenzen und Übertragung in Mikroorganismen geeignet ist. Er unterscheidet sich von dem Vektor pFDX3840 und pFDX3842 durch eine geringere Auswahl an Erkennungsstellen für singuläre Restriktionsendonukleasen (Polylinker) zur Klonierung von eukaryontischer DNA.

## Patentansprüche

1. Verfahren zur Mutagenese eukaryontischer Nukleotidsequenzen, **dadurch gekennzeichnet, daß**
a) eine genomische Nukleotidsequenz und/oder cDNA-Sequenz aus Eukaryonten in einen Mikroorganismus übertragen wird,
b) die eukaryontische DNA in dem Mikroorganismus durch sequenzunabhängige Insertionsmutagenese genetisch verändert wird,
c) die genetisch veränderte eukaryontische Nukleotidsequenz aus dem Mikroorganismus isoliert wird.

2. Verfahren gemäß Anspruch 1, **dadurch gekennzeichnet, daß** die sequenzunabhängige Insertionsmutagenese über die Ausbildung eines Kointegrats während der Konjugation zwischen zwei Mikroorganismen erfolgt.

3. Verfahren gemäß einem der Ansprüche 1 oder 2, **dadurch gekennzeichnet, daß** die eukaryontische DNA aus Pflanzen, Algen und/oder Pilzen stammt.

4. Verfahren gemäß einem der Ansprüche 1 bis 3, **dadurch gekennzeichnet, daß** die Mutagenese mit hoher Effizienz, bevorzugt mit einer relativen Frequenz nach der Selektion in einem Bereich von etwa 90 bis 100%, besonders bevorzugt von größer 90 bis 99%, insbesondere von 99,9% erfolgt.

5. Verfahren gemäß einem der Ansprüche 1 bis 4, **dadurch gekennzeichnet, daß** ein Vektor enthaltend eine eukaryontische Nukleotidsequenz und zusätzliche funktionelle Nukleotidsequenzen, bevorzugt mit Eigenschaften gemäß Fig. 1, Fig. 2, Fig. 3, Fig. 5 oder Fig. 6 verwendet und in einen Mikrooganismus übertragen wird.

6. Verfahren gemäß einem der Ansprüche 1 bis 5, **dadurch gekennzeichnet, daß** bezogen auf einen ausgewählten Bereich einer eukaryontischen Nukleotidsequenz durch die sequenzunabhängige Mutagenese eine Population verschiedenartiger eukaryontischer Nukleotidsequenzen erzeugt wird.

7. Verfahren gemäß einem der Ansprüche 1 bis 6, **dadurch gekennzeichnet, daß** die Mutagenese durch Insertion einer zellfremden Nukleotidsequenz, insbesondere prokaryontischen Ursprungs, erfolgt.

8. Verfahren gemäß einem der Ansprüche 1 bis 7, **dadurch gekennzeichnet, daß** es sich bei der zellfremden Nukleotidsequenz um ein Transposon, bevorzugt das Transposon Mini-Tn1000::nptll, handelt.

9. Verfahren gemäß einem der Ansprüche 1 bis 8, **dadurch gekennzeichnet, daß** ein Mikroorganismus eingesetzt wird, der die notwendigen Eigenschaften zur Transposition und Konjugation aufweist.

10. Verfahren gemäß einem der Ansprüche 1 bis 9, **dadurch gekennzeichnet, daß** als Mikroorganismus ein Bakterium der Gattung Enterobacteriaceae oder Bacillaceae eingesetzt wird.

11. Verfahren zur Herstellung genetisch veränderter eukaryontischer Zellen der Gattung Physcomitrella oder deren Nachkommen, **dadurch gekennzeichnet, daß**
a) in eine eukaryontische Wirtszelle eine gemäß Verfahren nach einem der Ansprüche 1 bis 10 genetisch veränderte eukaryontische Nukleotidsequenz übertragen wird,
b) diese transformierte eukaryontische Wirtszelle unter Bedingungen inkubiert wird, die eine gezielte homologe Rekombination der genetisch veränderten eukaryontischen Nukleotidsequenz aus Schritt a) in das Genom der Wirtszelle ermöglichen und/oder auslösen,
c) anschließend diejenigen eukaryontischen Wirtszellen identifiziert werden, die eine genetisch veränderte eukaryontische Nukleotidsequenz aus Schritt a) in ihrem Genom integriert enthalten und
d) aus diesen Zellen entsprechendes Gewebe von Pflanzen und/oder ganze Pflanzen regeneriert werden.

12. Verfahren gemäß Anspruch 11, **dadurch gekennzeichnet, daß** die homologe Rekombination der genetisch veränderten eukaryontischen Nukleotidsequenz in das Genom der eukaryontischen Wirtszelle der Gattung Physcomitrella zu einer phänotypisch sichtbar und/oder meßbar veränderten Erscheinungsform in der Zelle und/oder dem Gewebe von Pflanzen und/oder einer ganzen Pflanze führt.

13. Verfahren gemäß einem der Ansprüche 11 oder 12, **dadurch gekennzeichnet, daß**- die homologe Rekombination mit einer hohen Effizienz, bevorzugt mit einer relativen Frequenz nach der Selektion in einem Bereich von etwa 0,1 bis 99,9 %, besonders bevorzugt von 1 bis 90 %, insbesondere von größer als 10 % erfolgt.

14. Verfahren gemäß einem der Ansprüche 11 bis 13, **dadurch gekennzeichnet, daß** eine Population genetisch veränderter eukaryontischer Zellen der Gattung Physcomitrella erzeugt wird, deren Genom mit eine statistischen Wahrscheinlichkeit im Bereich von 90-100%, bevorzugt von 90-99%, besonders bevorzugt von 99,9%, an unterschiedlichen Stellen eine Veränderung trägt.

15. Verfahren gemäß einem der Ansprüche 11 bis 14, **dadurch gekennzeichnet, daß** Zellen von Physcomitrella patens eingesetzt werden.

16. Genetisch veränderte eukaryontische Zelle der Gattung Physcomitrella oder deren Nachkommen hergestellt in einem Verfahren gemäß einem der Ansprüche 11 bis 15.

17. Zell-Gewebe, fortpflanzungsfähiges Material, Samen und/oder Sporen von einer genetisch veränderten eukaryontischen Zellen der Gattung Physcomitrella gemäß Anspruch 16.

18. Pflanze und/oder Pflanzenteile, enthaltend und/oder regeneriert aus genetisch veränderte(n) eukaryontische(n) Zellen der Gattung Physcomitrella gemäß Anspruch 16.

19. Verwendung genetisch veränderter eukaryontischer Zellen der Gattung Physcomitrella gemäß Anspruch 16 zur Identifizierung funktioneller Nukleotidsequenzen in eukaryontischen Zellen und/oder zur Charakterisierung von eukaryontischen Nukleotidsequenzen unbekannter Funktion.

## Claims

1. A method for the mutagenesis of eukaryotic nucleotide sequences, which comprises
a) transferring a genomic nucleotide sequence and/or cDNA sequence from eukaryotic organisms into a microorganism,
b) genetically modifying the eukaryotic DNA in the microorganism by sequence-independent insertion mutagenesis, and
c) isolating the genetically modified eukaryotic nucleotide sequence from the microorganism.

2. The method according to claim 1, wherein the sequence-independent insertion mutagenesis is effected via cointegrate formation during the conjugation between two microorganisms.

3. The method according to claim 1 or 2, wherein the eukaryotic DNA originates from plants, algae and/or fungi.

4. The method according to any of claims 1 to 3, wherein the mutagenesis is effected with high efficiency, preferably with a relative frequency post-selection in a range of from approximately 90 to 100%, especially preferably of greater than 90 to 99%, in particular 99.9%.

5. The method according to any of claims 1 to 4, wherein a vector comprising a eukaryotic nucleotide sequence and additional functional nucleotide sequences, preferably with characteristics as shown in Fig. 1, Fig. 2, Fig. 3, Fig. 5 or Fig. 6, is used and transferred into a microorganism.

6. The method according to any of claims 1 to 5, wherein, based on a selected region of a eukaryotic nucleotide sequence, a population of different eukaryotic nucleotide sequences is generated by sequence-independent mutagenesis.

7. The method according to any of claims 1 to 6, wherein the mutagenesis is effected by insertion of a heterologous nucleotide sequence, in particular of prokaryotic origin.

8. The method according to any of claims 1 to 7, wherein the heterologous nucleotide sequence is a transposon, preferably the transposon mini-Tn1000::nptII.

9. The method according to any of claims 1 to 8, wherein a microorganism is employed which has the necessary characteristics for transposition and conjugation.

10. The method according to any of claims 1 to 9, wherein the microorganism employed is a bacterium of the genus Enterobacteriaceae or Bacillaceae.

11. A method of generating genetically modified eukaryotic cells of the genus Physcomitrella or their progeny, which comprises
a) transferring, into a eukaryotic host cell, a eukaryotic nucleotide sequence genetically modified in accordance with methods according to any of claims 1 to 10,
b) incubating this transformed eukaryotic host cell under conditions which make possible and/or which trigger a targeted homologous recombination of the genetically modified eukaryotic nucleotide sequence of step a) into the genome of the host cell,
c) subsequently identifying those eukaryotic host cells which comprise integrated in their genome a genetically modified eukaryotic nucleotide sequence of step a), and
d) regenerating, from these cells, corresponding tissue of plants and/or intact plants.

12. The method according to claim 11, wherein the homologous recombination of the genetically modified eukaryotic nucleotide sequence into the genome of the eukaryotic host cell of the genus Physcomitrella brings about a phenotypically visible and/or measurably modified phenotype in the cell and/or the tissue of plants and/or an intact plant.

13. The method according to claim 11 or 12, wherein the homologous recombination is effected with a high efficiency, preferably with a relative frequency post-selection in a range of from approximately 0.1 to 99.9%, especially preferably from 1 to 90%, in particular greater than 10%.

14. The method according to any of claims 11 to 13, wherein a population of genetically modified eukaryotic cells of the genus Physcomitrella is generated whose genome bears a modification at different sites with a statistic probability in the range of from 90-100%, preferably of 90-99%, especially preferably of 99.9%.

15. The method according to any of claims 11 to 14, wherein cells of Physcomitrella patens are employed.

16. A genetically modified eukaryotic cell of the genus Physcomitrella or its progeny, generated by a method according to any of claims 11 to 15.

17. Cell tissue, reproducible material, seeds and/or spores of a genetically modified eukaryotic cell of the genus Physcomitrella according to claim 16.

18. A plant and/or plant part comprising and/or regenerated from (a) genetically modified eukaryotic cell(s) of the genus Physcomitrella according to claim 16.

19. The use of genetically modified eukaryotic cells of the genus Physcomitrella according to claim 16 for identifying functional nucleotide sequences in eukaryotic cells and/or for characterizing eukaryotic nucleotide sequences with an unknown function.

## Revendications

1. Procédé de mutagenèse de séquences nucléotidiques eucaryotes, **caractérisé en ce que**
a) une séquence nucléotidique génomique et/ou une séquence d'ADNc provenant d'eucaryotes sont transférées dans un microorganisme,
b) l'ADN eucaryote se trouvant dans le microorganisme est soumis à une modification génétique par une mutagenèse par insertion indépendante de la séquence,
c) la séquence nucléotidique eucaryote génétiquement modifiée est isolée du microorganisme.

2. Procédé selon la revendication 1, **caractérisé en ce que** la mutagenèse par insertion, indépendante de la séquence, s'effectue par formation d'un co-intégrat pendant la conjugaison entre deux microorganismes.

3. Procédé selon l'une des revendications 1 ou 2, **caractérisé en ce que** l'ADN eucaryote provient de végétaux, d'algues et/ou de champignons.

4. Procédé selon l'une des revendications 1 à 3, **caractérisé en ce que** la mutagenèse est réalisée avec un rendement élevé, de préférence avec une fréquence relative, après la sélection, comprise dans la plage d'environ 90 à 100 %, d'une manière particulièrement préférée supérieure à 90 et allant jusqu'à 99 %, et en particulier de 99,9 %.

5. Procédé selon l'une des revendications 1 à 4, **caractérisé en ce qu'**on utilise un vecteur contenant une séquence nucléotidique eucaryote et des séquences nucléotidiques fonctionnelles supplémentaires, ayant de préférence les propriétés selon la Figure 1, la Figure 2, la Figure 3, la Figure 5 ou la Figure 6, et on transfère ce vecteur dans un microorganisme.

6. Procédé selon l'une des revendications 1 à 5, **caractérisé en ce que**, en liaison avec une zone sélectionnée d'une séquence nucléotidique eucaryote, on produit par mutagenèse indépendante de la séquence une population de séquences nucléotidiques eucaryotes de différents types.

7. Procédé selon l'une des revendications 1 à 6, **caractérisé en ce que** la mutagenèse est réalisée par insertion d'une séquence nucléotidique non cellulaire, en particulier d'origine procaryote.

8. Procédé selon l'une des revendications 1 à 7, **caractérisé en ce que**, pour ce qui concerne la séquence nucléotidique non cellulaire, il s'agit d'un transposon, de préférence du transposon mini-Tn1000::nptII.

9. Procédé selon l'une des revendications 1 à 8, **caractérisé en ce qu'**on utilise un microorganisme qui présente les propriétés nécessaires à une transposition et une conjugaison.

10. Procédé selon l'une des revendications 1 à 9, **caractérisé en ce qu'**on utilise en tant que microorganisme une bactérie du genre *Enterobacteriaceae* ou *Bacillaceae*.

11. Procédé de préparation de cellules eucaryotes génétiquement modifiées du genre *Physcomitrella* ou de leur descendance, **caractérisé en ce que**
a) on transfère dans une cellule hôte eucaryote une séquence nucléotidique eucaryote génétiquement modifiée par un procédé selon l'une des revendications 1 à 10,
b) on incube cette cellule hôte eucaryote transformée, dans des conditions qui permettent et/ou déclenchent une recombinaison homologue ciblée de la séquence nucléotidique eucaryote génétiquement modifiée de l'étape a) dans le génome de la cellule hôte,
c) puis on identifie les cellules hôtes eucaryotes qui contiennent, intégrée dans leur génome, une séquence nucléotidique eucaryote génétiquement modifiée de l'étape a), et
d) à partir de ces cellules, on régénère le tissu correspondant de végétaux, et/ou des plantes entières.

12. Procédé selon la revendication 11, **caractérisé en ce que** la recombinaison homologue de la séquence nucléotidique eucaryote génétiquement modifiée dans le génome de la cellule hôte eucaryote du genre *Physcomitrella* conduit à un phénotype modifié, visible et/ou mesurable d'un point de vue phénotypique, dans la cellule et/ou le tissu de végétaux et/ou d'une plante entière.

13. Procédé selon l'une des revendications 11 ou 12, **caractérisé en ce que** la recombinaison homologue est réalisée avec un rendement élevé, de préférence avec une fréquence relative, après la sélection, comprise dans la plage d'environ 0,1 à 99,9 %, d'une manière particulièrement préférée de 1 à 90 %, et en particulier supérieure à 10 %.

14. Procédé selon l'une des revendications 11 à 13, **caractérisé en ce qu'**on produit une population de cellules eucaryotes génétiquement modifiées du genre *Physcomitrella,* dont le génome porte en différents points une modification avec une probabilité statistique comprise dans la plage de 90 à 100 %, de préférence de 90 à 99 % et d'une manière particulièrement préférée de 99,9 %.

15. Procédé selon l'une des revendications 11 à 14, **caractérisé en ce qu'**on utilise des cellules de *Physcomitrella patens.*

16. Cellule eucaryote génétiquement modifiée du genre *Physcomitrella,* ou sa descendance, produite par un procédé selon l'une des revendications 11 à 15.

17. Tissu cellulaire, matériau apte à la reproduction, graine et/ou spores d'une cellule eucaryote génétiquement modifiée du genre *Physcomitrella* selon la revendication 16.

18. Plante et/ou parties de plante contenant une ou plusieurs cellules eucaryotes génétiquement modifiées du genre *Physcomitrella* selon la revendication 16, et/ou régénérées à partir de cette ou de ces cellules.

19. Utilisation de cellules eucaryotes génétiquement modifiées du genre *Physcomitrella* selon la revendication 16 pour l'identification de séquences nucléotidiques fonctionnelles dans des cellules eucaryotes et/ou pour la caractérisation de séquences nucléotidiques eucaryotes ayant une fonction inconnue.
